# EUROPÄISCHE PATENTSCHRIFT

(11) **EP 4 169 399 B1**
(45) Veröffentlichungstag und Bekanntmachung des Hinweises auf die Patenterteilung: **23.07.2025**
(21) Anmeldenummer: 21203534.9
(22) Anmeldetag: 19.10.2021
(51) Int. Cl.: A24F 40/42, A61M 11/04, A61M 15/06

(54) **SPENDER ZUR ABGABE EINER NIKOTIN- UND/ODER CANNABISHALTIGEN FLÜSSIGKEIT**
DISPENSER FOR DISPENSING A LIQUID CONTAINING NICOTINE AND / OR CANNABIS
DISTRIBUTEUR D'UN LIQUIDE CONTENANT DE LA NICOTINE ET/OU DU CANNABIS

(43) Veröffentlichungstag der Anmeldung: 26.04.2023
(73) Patentinhaber: Aptar Radolfzell GmbH, 78315 Radolfzell (DE)
(72) Erfinder: Alagia, Pierluigi, 78345 Moos (DE); Greiner-Perth, Jürgen, 78244 Gottmadingen (DE)
(74) Vertreter: Witte, Weller und Partner Patentanwälte mbB Stuttgart

(56) Entgegenhaltungen:
- WO-A1-2017/191205
- WO-A1-2019/224531
- DE-U1- 202019 005 638
- US-A1- 2002 008 122

## Beschreibung

### ANWENDUNGSGEBIET UND STAND DER TECHNIK

Die Erfindung betrifft Flüssigkeitsspender zur Abgabe einer Flüssigkeit, insbesondere einer nikotin- und/oder cannabishaltigen Flüssigkeit laut Ansprüche 1 bis 14.

Derartige Flüssigkeitsspender ersetzen den Konsum von Tabakprodukten. Statt Tabak oder andere Feststoffe zu verbrennen, wird nikotin- und/oder cannabishaltige Flüssigkeit in zerstäubter Form vom Nutzer aufgenommen. Gattungsgemäße Flüssigkeitsspender werden von ihren Nutzern im Alltag regelmäßig verwendet. Es ist wünschenswert, dass sie auf intuitive Art und Weise betätigt werden können.

### AUFGABE UND LÖSUNG

Aufgabe der Erfindung ist es primär, bauliche Möglichkeiten zur Realisierung eines solchen Spenders für den Austrag von nikotin- und/oder cannabishaltigen Flüssigkeiten zur Verfügung zu stellen, insbesondere einen Spender mit einem zuverlässigen und kostengünstig herstellbaren Auslassventil. Zur Lösung dieser Aufgabe wird ein Flüssigkeitsspender vorgeschlagen mit einem Flüssigkeitsspeicher zur Lagerung der Flüssigkeit vor dem Austrag und einem Mundstück, durch welches die Flüssigkeit abgegeben werden kann. Zwischen dem Flüssigkeitsspeicher und dem Mundstück erstreckt sich ein Flüssigkeitskanal, durch den hindurch Flüssigkeit zum Mundstück gelangen kann. Der Flüssigkeitskanal ist mit einer Auslassventilanordnung versehen, mit der der Flüssigkeitskanal geschlossen und geöffnet werden kann.

Insbesondere kann ein erfindungsgemäßer Flüssigkeitsspender eine in Richtung einer Haupterstreckungsrichtung erstreckte längliche und schlanke Formgebung aufweisen und ähnlich einer Zigarette gehandhabt werden. Das Mundstück und der vorzugsweise gegenüberliegend hierzu vorgesehene Flüssigkeitsspeicher erstrecken sich vorzugsweise jeweils in Richtung dieser Haupterstreckungsrichtung. Eine Außenseite des Flüssigkeitsspenders kann eine einheitliche zylindrische Formgebung aufweisen. Vorzugsweise ist der Flüssigkeitsspenders im Bereich des Flüssigkeitsspeichers jedoch mit einem größeren Querschnitt als im Bereich des Mundstücks ausgebildet.

Das Mundstück wird vom Nutzer mit den Lippen umschlossen oder den Zähnen erfasst, wenn Flüssigkeit ausgetragen werden soll. Das Mundstück weist vorzugsweise eine in etwa röhrenförmige Gestalt mit einer endseitigen Auslassöffnung auf. Die Auslassöffnung weist vorzugsweise einen lichten Querschnitt von mindestens 20 mm² auf. Innerhalb des Mundstücks wird die Flüssigkeit zugeführt, die dabei insbesondere vorzugsweise zum Zwecke der Einnahme zerstäubt wird. Vorzugsweise ist das Mundstück über einen weiteren Kanal mit der Umgebung verbunden, so dass zusammen mit der Flüssigkeit auch Luft vom Nutzer eingesogen werden kann. Dieser Lufteinlasskanal ist vorzugsweise als radial ausgerichtete Öffnung in einer Wandung des Mundstücks vorgesehen.

Der Flüssigkeitsspeicher ist im verkaufsfertigen Zustand mit der austragenden Flüssigkeit befüllt, also insbesondere mit einer nikotin- und/oder cannabishaltigen Flüssigkeit. Das Volumen des Flüssigkeitsspeichers liegt vorzugsweise bei weniger als 100 ml, insbesondere vorzugsweise bei weniger als 30 ml.

Gemäß zwei Aspekten der Erfindung werden verschiedene Gestaltungen der Auslassventilanordnung vorgeschlagen, die jedoch auch gemeinsam realisiert sein könnten.

Gemäß einem ersten Aspekt der Erfindung ist vorgesehen, dass die Auslassventilanordnung einen elastisch verformbaren Schlauchabschnitt aufweist, der zumindest einen Teil des Flüssigkeitskanals bildet.

Um die Auslassventilanordnung öffnen und schließen zu können, ist eine Betätigungsfläche an einer Außenseite des Spenders vorgesehen, insbesondere zur manuellen Betätigung oder zur Betätigung mit dem Mund. Diese Betätigungsfläche ist gegenüber einem den Flüssigkeitskanal umgebenden Gehäuseteil und/oder gegenüber dem Flüssigkeitsspeicher beweglich und mit einem Schlauchformungssegment gekoppelt, welches eine durch den Nutzer herbeigeführte Verlagerung der Betätigungsfläche in eine Verformung oder Rückverformung des Schlauchabschnitts wandelt. Durch Kraftbeaufschlagung der Betätigungsfläche kann der Schlauchabschnitt somit aus einem den Flüssigkeitskanal verschließenden Zustand in einen den Flüssigkeitskanal öffnenden Zustand verformt werden. Vorzugsweise ist eine Rückstellfeder vorgesehen, durch die die Betätigungsfläche in Richtung ihrer unbetätigten Endlage kraftbeaufschlagt ist und gegen deren Kraft die Betätigung der Betätigungsfläche stattfindet.

Zwei unterschiedliche Konzepte zur Verwendung des Schlauchabschnitts als Teil der Auslassventilanordnung werden bevorzugt. Das erste der Konzepte basiert auf einem Zusammendrücken des Schlauchs, das zweite der Konzepte basiert auf einem Abknicken des Schlauchs.

Beim ersten Konzept ist das Schlauchformungssegment orthogonal zum Schlauchabschnitt verlagerbar. Es kann bei einer möglichen Gestaltung ausschließlich orthogonal verlagerbar sein, also eine Verlagerungsrichtung aufweisen, die im 90°-Winkel zur Erstreckungsrichtung des Schlauchabschnitts ausgerichtet ist. Die Verlagerbarkeit des Schlauchformungssegments kann auch eine orthogonale Komponente aufweisen, gleichzeitig aber auch parallel zur Erstreckungsrichtung des Schlauchabschnitts verlagerbar sein.

Die Verlagerung des Schlauchformungssegments erfolgt zwischen einer schließenden Endlage und einer öffnenden Endlage. In der schließenden Endlage drückt das Schlauchformungssegment von außen gegen den Schlauchabschnitt und drückt diesen somit gegen eine Gegenfläche, insbesondere gegen eine ortsfeste Haltefläche, beispielsweise eine Haltefläche an einem inneren Umfang eines den Schlauchabschnitt partiell umhüllenden Rohrabschnitts. Der lichte Querschnitt des Schlauchabschnitts wird hierdurch weitgehend oder sogar auf Null verjüngt.

In der öffnenden Endlage ist das Schlauchformungssegment vom Schlauchabschnitt weg verlagert, so dass der elastische Schlauchabschnitt sich unter dem Eindruck des Flüssigkeitsdrucks wieder entspannten kann und den Flüssigkeitsstrom durch den Schlauchabschnitt zulässt.

Die Betätigungsfläche wird vorzugsweise zum Zwecke des Austrags in Richtung eines Zentrums oder einer Mittelachse eingedrückt. Um hierdurch eine Bewegung des Schlauchformungssegments vom Schlauchabschnitt weg zu wirken, kann eine Bewegungsrichtungsumkehr zwischen der Betätigungsfläche und dem Schlauchformungssegment vorgesehen sein, so dass das Schlauchformungssegment in einer Bewegungsrichtung verlagert wird, die von der Bewegungsrichtung der Betätigungsfläche abweicht. Derartiges ist beispielsweise einfach über ein Kippelement erzielbar, das auf gegenüberliegenden Seiten einer Kippachse das Schlauchformungssegment und die Betätigungsfläche aufweist.

Eine andere mögliche Gestaltung sieht vor, dass das Schlauchformungssegment und die Betätigungsfläche auf einander gegenüberliegenden Seiten des Schlauchabschnitts vorgesehen sind. Die Betätigungsfläche und das Schlauchformungssegment werden dann in etwa in gleicher Richtung verlagert, vorzugweise in exakt gleicher Richtung und gleichem Maße. Der Verbund von Betätigungsfläche und Schlauchformungssegment umgreift bei einer solchen Gestaltung den Schlauchabschnitt.

Beim zweiten Konzept basiert das Schließen der Auslassventilanordnung auf einem Abknicken des Schlauchs. Das Schlauchformungssegment ist hierbei verlagerbar zwischen einer schließenden Endlage und einer öffnenden Endlage, vorzugsweise durch feste oder sogar einstückige Verbindung des Schlauchformungssegments mit der Betätigungsfläche.

In der schließenden Endlage zwingt das Schlauchformungssegment den Schlauchabschnitt in einen geschlossenen Zustand, in dem der Schlauchabschnitt in mindestens einem Knickbereich mindestens einmal geknickt ist. Der Schlauchabschnitt ist dabei soweit geknickt, dass einander gegenüberliegende Innenflächen des Schlauchabschnitts im Knickbereich dichtend aneinander anliegen. Der beispielsweise in entspanntem Zustand runde Querschnitt des Schlauchs ändert in Richtung des Knickbereichs seine Formgebung hin zu einer zunächst elliptischen Form, die an der eigentlichen Knickstelle sich linienförmig verengt und hierdurch den Querschnitt auf Null sinken lässt. Diese Knickstelle bildet die Trennstelle des Auslassventils. Der Schlauchabschnitt knickt in der Knickstelle vorzugsweise um mindestens 90° ab, insbesondere um mindestens 105°. Der engste Krümmungsradius an einer Außenseite des Schlauchabschnitts an der Knickstelle ist vorzugsweise kleiner als der dreifache Durchmesser des Schlauchabschnitts, insbesondere vorzugsweise kleiner als der zweifache Durchmesser des Schlauchabschnitts.

Sobald das Schlauchformungssegment in seine öffnende Endlage zurückgekehrt ist, kehrt der Krümmungsbereich in einen entspannteren Zustand des Schlauchabschnitts zurück, in dem sich der lichte Querschnitt des Schlauchabschnitts an der Knickstelle wieder öffnet. Nun kann der Schlauchabschnitt wieder von Flüssigkeit durchströmt werden. Im geöffneten Zustand weichen die Schlauchabschnittsrichtungen beidseitig der Knickstelle vorzugsweise um weniger als 90° voneinander ab, insbesondere vorzugsweise um weniger als 75°.

Ob Flüssigkeit durch einen geknickten Schlauchabschnitt hindurchtreten kann, hängt auch vom Flüssigkeitsdruck ab. Es kann von Vorteil sein, wenn der Schlauchabschnitt mehrere Knickbereiche, insbesondere zwei Knickbereiche aufweist, an denen der lichte Innenquerschnitt des Schlauchabschnitts an der Knickstelle sich jeweils auf Null oder nahe Null verengt. Solche zwei oder mehr Knickbereiche, die bei Niederdrücken der Betätigungsfläche gleichzeitig verengt werden, können gemeinsam die Ventilwirkung erzielen, indem ein erster Knickbereich zwar noch von unter hohem Druck anstehender Flüssigkeit durchströmbar bleibt, dabei jedoch den Druck ausreichend absenkt, dass die Knickstelle im zweiten Knickbereich den Durchfluss vollständig unterbinden kann.

Bei mehr als einer Knickstelle ist es von besonderem Vorteil, wenn die der Schlauchabschnitt in den beiden Knickbereichen in entgegengesetzte Richtungen knickt, also in Art einer Z-Form. Eine solche Z-Form gestattet es, dass die angrenzenden Schlauchteile außenseitig der Knickbereiche eine identische Erstreckungsrichtung aufweisen können und durch ein Verbindungssegment verbunden sein können, welches mit den beidseitigen Schlauchteilen jeweils einen Knickbereich bilden. Allerdings sind die angrenzenden Schlauchteile in einem solchen Falle gegeneinander versetzt.

Um dies zu verhindern, kann es von Vorteil sein, wenn der Schlauchabschnitt insgesamt vier Knickbereiche aufweist, die die Ecken einer Parallelogramm-Form bilden und von denen zwei bei Anordnung des Schlauchformungssegments in der schließenden Endlage flüssigkeitsdichtend geknickt sind, also im Bereich ihrer jeweiligen Knickstelle einen lichten Durchmesser von Null aufweisen. Die anderen beiden Knickbereiche schließen nicht, wenn das Schlauchformungssegment in seine schließende Endlage gedrückt wird, sondern verringern ihren Knickwinkel.

Das Schlauchformungssegment kann je nach Art der zum Öffnen der Auslassventilanordnung erforderlichen Verformung des Schlauchabschnitts von diesem getrennt sein und nur im Falle der Kraftbeaufschlagung der Betätigungsfläche gegen den Schlauch gedrückt werden. Das Schlauchformungssegment kann jedoch auch einen Kopplungsabschnitt zur dauerhaften Ankopplung an den Schlauchabschnitt aufweisen, beispielsweise in Form einer seitlich geschlitzten Schlauchklemme, in die der Schlauchabschnitt eingesetzt ist. Eine solche Kopplung ist sinnvoll, um den Schlauchabschnitt sowohl bei der Überführung in den geschlossenen Zustand als auch bei der Überführung in den geöffneten Zustand sicher verlagern zu können, ohne hierfür die inhärente Rückstellneigung des Schlauchabschnitts oder die durch den Flüssigkeitsdruck bewirkte Rückstellneigung nutzen zu müssen.

Ein Flüssigkeitsspender der beschriebenen Art wird vom Nutzer bei Bedarf genutzt, üblicherweise mehrmals täglich. Der Nutzer führt den Flüssigkeitsspender zur Nutzung an die richtige Stelle, also insbesondere an den Mund, und drückt dann auf die Betätigungsfläche.

Bei diesem Druck kann es sich bei einer Ausgestaltung um einen manuell aufgebrachten Druck handeln. Der Nutzer drückt also beispielsweise mit dem Zeigefinger auf die Betätigungsfläche an einer hierfür vorgesehenen Taste oder einem Schieber. Die Betätigungsfläche wird gegenüber dem Gehäuse verlagert und die Auslassventilanordnung hierdurch geöffnet.

Vorzugsweise ist die Betätigungsfläche zum Öffnen der Auslassventilanordnung bezogen auf die Haupterstreckungsrichtung des Flüssigkeitsspenders oder eines zylindrischen Teilabschnitts hiervon radial eindrückbar. Diese Bauweise eignet sich insbesondere für einen sehr einfachen Aufbau mit einem den Schlauchabschnitt schließend zusammendrückenden Schlauchformungssegment, welches durch Kraftbeaufschlagung der Betätigungsfläche vom Schlauchabschnitt beabstandet wird. Eine andere bevorzugte Gestaltung sieht vor, dass die Betätigungsfläche, die hierfür vorzugsweise an einem hülsenartigen Bauteil vorgesehen ist, zum Öffnen der Auslassventilanordnung axial verlagerbar ist.

Neben der manuellen Betätigung wird die Betätigung mit dem Mund als zweckmäßig angesehen. Hierbei übt der Nutzer mit seinen Lippen oder seinen Zähnen einen Druck auf das Mundstück aus, wobei es auch zu einer Kraftbeaufschlagung der Betätigungsfläche kommt.

Die Betätigungsfläche ist dafür vorzugsweise im Bereich des Mundstücks angeordnet und durch die Lippen oder Zähne des Benutzers niederdrückbar. Die erforderliche Kraft sollte hierbei möglichst unterhalb von 5 Newton liegen.

Die Betätigungsfläche kann gegenüber dem Mundstück, insbesondere gegenüber einer gegenüberliegenden Wandung des Mundstücks, niedergedrückt werden. Insbesondere kann die Betätigungsfläche dabei schwenkbeweglich ausgestaltet sein.

Die Betätigungsfläche kann jedoch auch mit dem gesamten Mundstück einstückig sein bzw. durch das gesamte Mundstück gebildet sein. Dieses Mundstück ist dann als Ganzes bezogen auf eine Haupterstreckungsrichtung gegenüber dem Flüssigkeitsspeicher oder anderen Gehäuseabschnitten des Flüssigkeitsspenders verlagerbar, insbesondere axial verlagerbar.

Ein Flüssigkeitsspender der beschriebenen Art ist vorzugsweise mit einem Druckspeicher versehen, so dass die Flüssigkeit bei geöffneter Auslassventilanordnung selbsttätig ausströmt.

Insbesondere kann der Flüssigkeitsspeicher partiell wechselbar sein. Hierzu wird insbesondere vorgeschlagen, dass der Flüssigkeitsspeicher eine Aufnahme zur Aufnahme einer wechselbaren Flüssigkeitskartusche aufweist. In diese Aufnahme wird eine Flüssigkeitskartusche vorzugsweise werkzeuglos eingesetzt. Ist eine eingesetzte Kartusche leer, so kann der Nutzer diese von der Aufnahme trennen und durch eine neue ersetzen.

Flüssigkeitskartuschen der beschriebenen Art verfügen vorzugsweise über ein zusätzliches Ventil, durch das ihr Speicherraum bis zur Verwendung von einer Umgebung getrennt ist. Dieses zusätzliche Ventil kann insbesondere durch Einsetzen der Flüssigkeitskartusche in die Aufnahme geöffnet werden. Insbesondere weisen die Flüssigkeitskartuschen einen Auslassstutzen auf, der beim Einsetzen in der Aufnahme gegenüber einer Speicherwandung der Flüssigkeitskartusche eingedrückt oder verkippt wird und dadurch das zusätzliche Ventil öffnet.

Der Flüssigkeitsspender gemäß dem zweiten Aspekt der Erfindung weist ebenfalls einen Flüssigkeitsspeicher zur Lagerung der Flüssigkeit vor dem Austrag, ein Mundstück auf, durch welches die Flüssigkeit abgegeben werden kann, sowie einen verbindenden Flüssigkeitskanal, der mittels einer Auslassventilanordnung geschlossen und geöffnet werden kann. Alle hierzu vorgenannten Merkmale können auch beim Flüssigkeitsspender gemäß diesem zweiten Aspekt der Erfindung einzeln oder in Kombination verwirklicht sein.

Die Auslassventilanordnung weist im Falle dieses zweiten Erfindungsaspekts ein Kippventil auf, welches über einen kippbeweglichen Auslassstutzen verfügt, der von dem Flüssigkeitskanal durchdrungen ist. In Abhängigkeit der Kippstellung des Auslassstutzens wird das Kippventil geöffnet bzw. geschlossen. Technisch kann ein Kippventil insbesondere derart umgesetzt sein, dass eine vorzugsweise umlaufende Ventilfläche mit dem Auslassstutzen verbunden ist, die durch die Kippbewegung von einer ebenfalls vorzugsweise umlaufenden Gegenfläche abgehoben wird und so einen Flüssigkeitsstrom zum Mundstück gestattet. Vorzugsweise kann der Auslassstutzen in beliebige Richtung verkippt werden, um geöffnet zu werden.

Ein Kippventil hat sich neben dem beschriebenen Schlauchventil als besonders vorteilhafte und kostengünstige Gestaltung einer Auslassventilanordnung erwiesen. Es kann bereits durch ein geringes Verkippen geöffnet werden und gestattet dennoch ein sehr dosiertes Öffnen und Schließen.

Die Betätigung eines solchen Kippventils kann im einfachsten Falle direkt über das Mundstück erfolgen. Das Mundstück ist hierfür gegenüber dem Flüssigkeitsspeicher beweglich ausgebildet und dabei mit dem Auslassstutzen derart gekoppelt, dass eine Bewegung des Mundstücks gegenüber dem Flüssigkeitsspeicher den Auslassstutzen verkippt. Erzielbar ist dies beispielsweise dadurch, dass das Mundstück fest mit dem Auslassstutzen verbunden ist und der Benutzer durch geringfügiges Anwinkeln des Mundstücks gegenüber dem Flüssigkeitsspender, vorzugsweise um einen Winkel von weniger als 10°, das Kippventil öffnet und den Flüssigkeitsaustrag damit ermöglicht.

Aber auch bei diesem Flüssigkeitsspender gemäß dem zweiten Aspekt der Erfindung ist es möglich, eine Betätigungsfläche an der Außenseite des Spenders getrennt vom Mundstück vorzusehen, die gegenüber dem Flüssigkeitsspeicher und/oder dem Mundstück beweglich ist, und die mit dem Auslassstutzen wirkgekoppelt ist, so dass durch Kraftbeaufschlagung der Betätigungsfläche der Auslassstutzen verkippt wird. Auch hier ist die manuelle Betätigung oder eine Betätigung mit dem Mund möglich, wobei die Betätigungsfläche je nach gewünschter Art näher am Mundstück oder weiter hiervon entfernt platziert wird.

Im einfachsten Falle kann die Betätigungsfläche orthogonal zum Auslassstutzen verlagerbar sein und im eingedrückten Zustand den Auslassstutzen in eine verkippte Stellung drücken.

Sofern beim Öffnen des Kippventils eine Verlagerung des Auslassstutzens gegenüber dem Mundstück vorgesehen ist, kann es zweckmäßen sein, zwischen dem Auslassstutzen und dem Mundstück einen Schlauchabschnitt vorzusehen, der den entstandenen Versatz ausgleichen kann.

Das Kippventil kann grundsätzlich getrennt von einer wechselbaren Flüssigkeitskartusche vorgesehen sein. Es wird jedoch bevorzugt, dass der Flüssigkeitsspeicher über eine wechselbare Flüssigkeitskartusche verfügt, an der das Kippventil unmittelbar vorgesehen ist. Mit dem Austausch der Flüssigkeitskartusche findet somit auch ein Austausch des Kippventils statt.

Der Flüssigkeitsspender ist vorzugsweise dafür ausgebildet, die Flüssigkeit in fein zerstäubter Form abzugeben. Dies ist beispielsweise über eine Zerstäubungseinrichtung mit Wirbelkammer am Ende des Flüssigkeitskanals erreichbar. Bevorzugt ist allerdings eine Gestaltung, bei der eine Austragdüseneinheit vorgesehen ist, durch die Flüssigkeit in das Mundstück abgegeben werden kann. Diese Austragdüseneinheit verfügt vorzugsweise über eine Mehrzahl von Düsenöffnungen, insbesondere über eine Düsenplatte, in der eine Mehrzahl von Düsenöffnungen vorgesehen sind. Die Düsenöffnungen können dabei insbesondere divergierend ausgerichtet sein. Eine solche Düsenplatte ermöglicht die Herstellung eines besonders feinen Tröpfchenstrahls auch bei insgesamt sehr geringem Volumenstrom, wie er bei den bevorzugten Anwendungsfeldern des Flüssigkeitsspenders gewünscht ist. Vorzugsweise weist die Düsenplatte mindestens 10 Düsenöffnungen auf. Der lichte Querschnitt der Düsenöffnungen an der engsten Stelle beträgt vorzugsweise weniger als 250 µm².

Der genannte Schlauchabschnitt bei der ersten Variante der Erfindung ist vorzugsweise aus einem elastischen Kunststoffmaterial gefertigt, insbesondere aus Polyethylen, aus Polypropylen oder aus einem Polyethylen und/oder Polypropylen enthaltenden Material. Da beim bevorzugten Anwendungsfeld des Austrags von nikotin- und/oder cannabishaltigen Flüssigkeiten nur geringe Flüssigkeitsströme erforderlich sind, weist der Schlauchabschnitt vorzugsweise einen Innendurchmesser von 2,0 mm oder weniger auf, insbesondere von 1,0 mm oder weniger. Weiterhin weist der Schlauchabschnitt vorzugsweise eine Wandungsstärke von 1,0 mm oder weniger auf, insbesondere vorzugsweise von 0,5 mm oder weniger. Vorzugsweise beträgt die Wandungsstärke 0,1 mm oder mehr, insbesondere 0,3 mm oder mehr. Vorzugsweise beträgt der Innendurchmesser zwischen 30% und 70% des Außendurchmessers des Schlauchabschnitts.

Wenngleich die beschriebenen Flüssigkeitsspender insbesondere für das genannte Anwendungsfeld des Austrags von nikotin- und/oder cannabishaltigen Flüssigkeiten vorgesehen sind, sind einige Aspekte der Erfindung auch getrennt hiervon realisierbar.

Einen separaten Erfindungsaspekt stellt eine Gestaltung eines Flüssigkeitsspenders dar, der einen als Druckspeicher ausgebildeten Flüssigkeitsspeicher zur Lagerung der Flüssigkeit vor dem Austrag und eine Abgabeöffnung aufweist, wobei die Abgabeöffnung nicht zwingend im Bereich eines Mundstücks angeordnet sein muss. Der Flüssigkeitsspeicher und die Abgabeöffnung sind miteinander über einen Flüssigkeitskanal verbunden, dem eine Auslassventilanordnung zugeordnet ist, mittels derer der Flüssigkeitskanal geschlossen und geöffnet werden kann. Wie oben zum zweiten Konzept der ersten Variante der Erfindung erläutert, weist die Auslassventilanordnung einen elastisch verformbaren Schlauchabschnitt auf, der einen Teil des Flüssigkeitskanals sowie zumindest einen Teil der Auslassventilanordnung bildet, die vorzugsweise vom Nutzer mittels einer Handhabe geschaltet werden kann.

In einem offenen Zustand der Auslassventilanordnung kann der Schlauchabschnitt von Flüssigkeit durchströmt werden. In einem geschlossenen Zustand ist der Schlauchabschnitt in mindestens einem Knickbereich mindestens einmal geknickt, wobei in der oben schon beschriebenen Weise vorzugsweise eine Mehrzahl von Knickstellen vorgesehen sind. An den Knickstellen liegen einander gegenüberliegende Innenflächen des Schlauchabschnitts dichtend aneinander an, so dass der Schlauchabschnitt nicht von Flüssigkeit durchströmt werden kann.

Dieses Grundprinzip des einfach oder mehrfach abgeknickten Schlauchabschnitts zur Bildung eines schaltbaren Auslassventils kann bei verschiedenen Flüssigkeitsspendern Anwendung finden. Insbesondere ist diese Technik bei den bereits beschriebenen Nikotin- bzw. Cannabisspendern verwendbar, jedoch auch zum Austrag eines flüssigen Medikaments, eines Mundsprays oder eines Zahnpflegeprodukts. Der Flüssigkeitsspeicher, der vorzugsweise als Druckspeicher ausgebildet ist, nimmt bei solchen Gestaltungen eine entsprechende Flüssigkeit auf.

Die oben beschriebenen weiteren möglichen Merkmale eines Nikotin- bzw. Cannabisspenders können einzeln oder in Kombination auch bei einem solchen Spender in einem anderen Anwendungsfeld Verwendung finden. WO2019/224531 beschreibt ein Nikotin Spender mit einem druckabhängig öffnendes Ventil, dass wirkt auf ein Flüssigkeitskanal.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1A und 1B zeigen in verallgemeinerter Form einen möglichen Grundaufbau eines erfindungsgemäßen Flüssigkeitsspenders.
Fig. 2A und 2B zeigen den Detailaufbau eines ersten Ausführungsbeispiels eines erfindungsgemäßen Flüssigkeitsspenders im unbetätigten und im betätigten Zustand.
Fig. 3A und 3B zeigen den Detailaufbau eines zweiten Ausführungsbeispiels eines erfindungsgemä-βen Flüssigkeitsspenders im unbetätigten und im betätigten Zustand.
Fig. 4A und 4B zeigen den Detailaufbau eines dritten Ausführungsbeispiels eines erfindungsgemä-βen Flüssigkeitsspenders im unbetätigten und im betätigten Zustand.
Fig. 5A und 5B zeigen den Detailaufbau eines vierten Ausführungsbeispiels eines erfindungsgemä-βen Flüssigkeitsspenders im unbetätigten und im betätigten Zustand.
Fig. 6A und 6B zeigen den Detailaufbau eines fünften Ausführungsbeispiels eines erfindungsgemä-βen Flüssigkeitsspenders im unbetätigten und im betätigten Zustand.
Fig. 7A und 7B zeigen den Detailaufbau eines sechsten Ausführungsbeispiels eines erfindungsgemä-βen Flüssigkeitsspenders im unbetätigten und im betätigten Zustand.
Fig. 8 bis 10 zeigen alternative Gestaltungen zu den Ausführungsbeispielen der Fig. 6A bis 7B.
Fig. 11A und 11B zeigen den Detailaufbau eines weiteren Ausführungsbeispiels eines erfindungsgemäßen Flüssigkeitsspenders im unbetätigten und im betätigten Zustand.
Fig. 12 und 13 zeigen alternative Gestaltungen zum Ausführungsbeispiel der Fig. 11A und 11B.

### DETAILLIERTE BESCHREIBUNG DER AUSFÜHRUNGSBEISPIELE

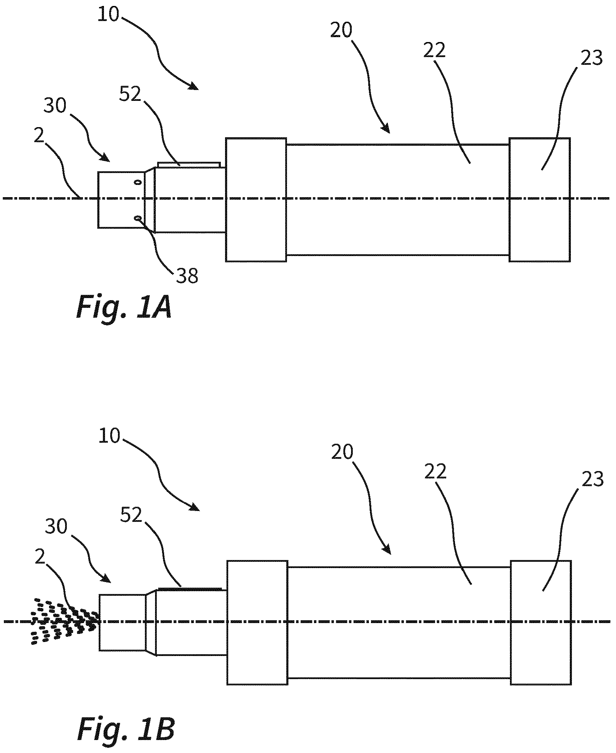

Die Fig. 1A und 1B zeigen zunächst eine bevorzugte Bauweise eines erfindungsgemäßen Flüssigkeitsspenders in ungeschnittener Darstellung. Ein solcher, insbesondere für den Austrag von nikotin- oder cannabishaltiger Flüssigkeit vorgesehener Flüssigkeitsspender 10 weist eine in Richtung einer Haupterstreckungsrichtung 2 ausgerichtete Formgebung auf. An einem proximalen Ende des Flüssigkeitsspenders 10 ist ein Mundstück 30 vorgesehen. An einem gegenüberliegenden distalen Ende ist ein Flüssigkeitsspeicher 20 vorgesehen, der beim Beispiel der Fig. 1A und 1B eine schachtförmige Aufnahme 22 aufweist, die von einem Verschluss 23 verschlossen ist und der Aufnahme einer in den Fig. 1A und 1B nicht ersichtlichen Druckspeicherkartusche dient. Zwischen dem Flüssigkeitsspeicher 20 und dem Mundstück30 ist ein in den Fig. 1A und 1B nicht dargestellter verbindender Flüssigkeitskanal 40 vorgesehen, der über eine Auslassventilanordnung 50 verfügt. Diese Auslassventilanordnung 50 gestattet es, den Flüssigkeitskanal 40 ausgehend von einem im Ruhezustand geschlossenen Zustand zu öffnen, beispielsweise mittels einer Betätigungsfläche 52. Ist die Auslassventilanordnung 50 geöffnet, so kann Flüssigkeit durch den Flüssigkeitskanal 40 zum Mundstück 30 strömen und dort in Form eines zerstäubten Sprühstrahls ausgetragen werden, wie in Fig. 1B verdeutlicht ist. Die derart versprühte oder vernebelte Flüssigkeit wird vom Nutzer eingesogen, wobei vorzugsweise zusätzlich Luft durch Lufteinlasskanäle 38 mit eingesogen wird.

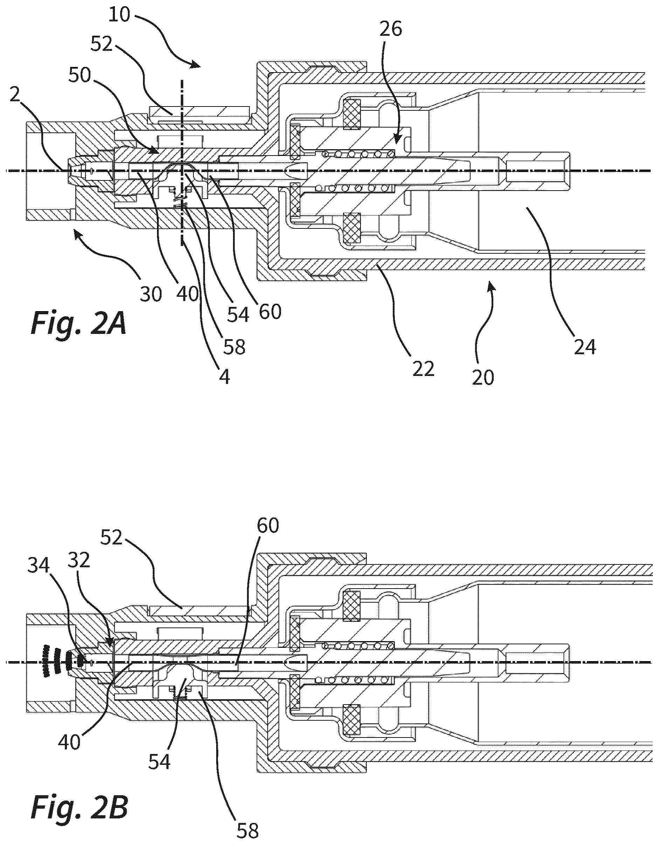

Die Fig. 2A und 2B zeigen ein erstes Ausführungsbeispiel in einer geschnittenen Darstellung. Hier ist zu erkennen, dass in der Aufnahme 22 des Flüssigkeitsspeichers 20 die bereits genannte Flüssigkeitskartusche 24 angeordnet ist, die über ein Auslassventil 26 verfügt. Ist die Flüssigkeitskartusche 24 im Flüssigkeitsspender 10 aufgenommen, so ist dieses Auslassventil 26 geöffnet.

Von einem Auslassstutzen 27 der Flüssigkeitskartusche 24 erstreckt sich ein rohrförmiger Gehäuseabschnitt, innerhalb dessen ein Schlauchabschnitt 60 platziert ist. Der den Schlauchabschnitt 60 umgebende rohrförmige Gehäuseabschnitt ist an seiner Unterseite mit einer Öffnung versehen. Hier befindet sich ein in Verlagerungsrichtung 4 verlagerbares Schlauchformungssegment 54, welches mittels einer Rückstellfeder 28 nach oben und somit gegen den Schlauchabschnitt 60 gedrückt wird.

In einem Ruhezustand des Spenders wird der Schlauchabschnitt 60 hiermit so weit zusammengedrückt, dass keine Flüssigkeit in Richtung des Mundstücks 30 und zu der dort angeordneten Austragdüseneinheit 32 strömen kann. Das genannte Schlauchformungssegment 54 ist beispielsweise mittels einer Rastverbindung mit einem Betätigungsbauteil verbunden, an dem auch die genannte Betätigungsfläche 52 vorgesehen ist. Auch eine einstückige Gestaltung ist möglich.

Wenn nun in der in Fig. 2B verdeutlichten Weise die Betätigungsfläche 52 manuell gegen die Kraft der Rückstellfeder 58 niedergedrückt wird, so entfällt damit ein Teil der auf den Schlauchabschnitt 60 wirkenden Kraft, so dass der Flüssigkeitskanal 40 geöffnet wird und Flüssigkeit zur Austragdüseneinheit 32 strömen kann. Hier wird die Flüssigkeit gegen eine mit feinen Durchbrechungen versehene Düsenplatte 34 gedrückt, welche die gewünschte Zerstäubung der Flüssigkeit bewirkt. Der Nutzer saugt während der Betätigung der Betätigungsfläche 52 an dem von seinen Lippen umschlossenen Mundstück 30, wobei neben der durch die Austragdüseneinheit 32 ausgetragenen Flüssigkeit auch Luft eingesogen wird, die durch einen Lufteinlasskanal 38 in das Mundstück einströmen kann.

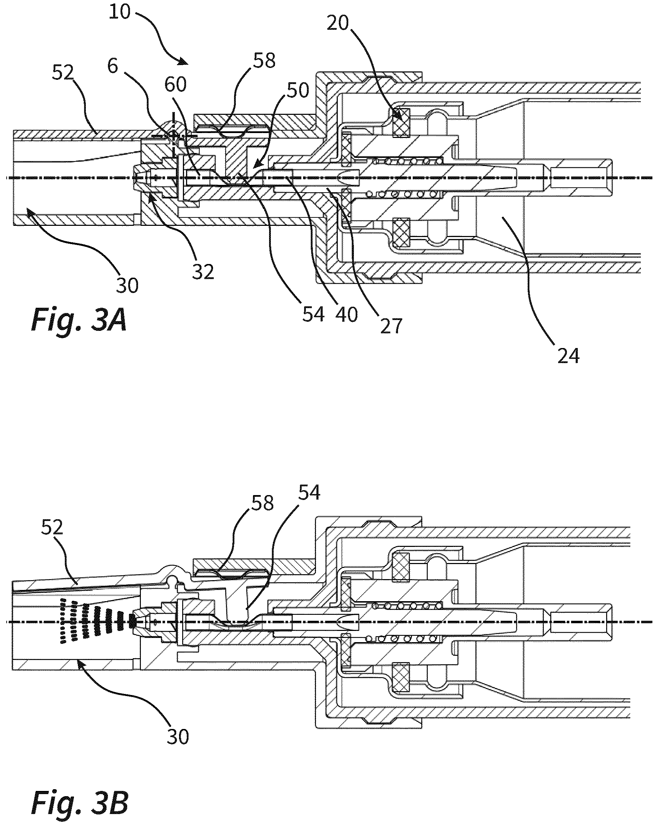

Bei dem Ausführungsbeispiel der Fig. 3A und 3B ist die grundsätzliche Funktionsweise sehr ähnlich. Auch hier ist zwischen einem Auslassstutzen 27 einer Flüssigkeitskartusche 24 und der Austragdüseneinheit 32 ein abschnittsweise durch einen Schlauchabschnitt 60 gebildeter Flüssigkeitskanal 40 vorgesehen. Der Schlauchabschnitt 60 wird auch hier durch ein Schlauchformungssegment 54 zusammengedrückt, wobei dieses abweichend von der Gestaltung der Fig. 2A und 2B von oben auf den Schlauchabschnitt 60 drückt. Das Schlauchformungssegment 54 ist bei dieser Gestaltung gemeinsam mit der Betätigungsfläche 52 Teil eines gemeinsamen Wippenelements, welches um die Schwenkachse 6 verschwenkbar ist.

Das Öffnen der Auslassventilanordnung 50 erfolgt beim Ausführungsbeispiel der Fig. 3A und 3B mit dem Mund. Die Betätigungsfläche 52 erstreckt sich daher bis in den Bereich des Mundstücks 30 und kann gemeinsam mit dem Mundstück 30 von den Lippen oder den Zähnen eines Nutzers umschlossen werden. Drückt der Nutzer seine Lippen oder Zähne zusammen, so verschwenkt das Wippenelement bezogen auf die Figuren gegen den Uhrzeigersinn und drückt somit das Schlauchformungssegment 54 gegen die Kraft der Rückstellfeder 58 nach oben. Hierdurch entfällt die den Schlauchabschnitt 60 zusammendrückende Kraft und es kommt zum Flüssigkeitsaustrag, wie in Fig. 3B verdeutlicht ist.

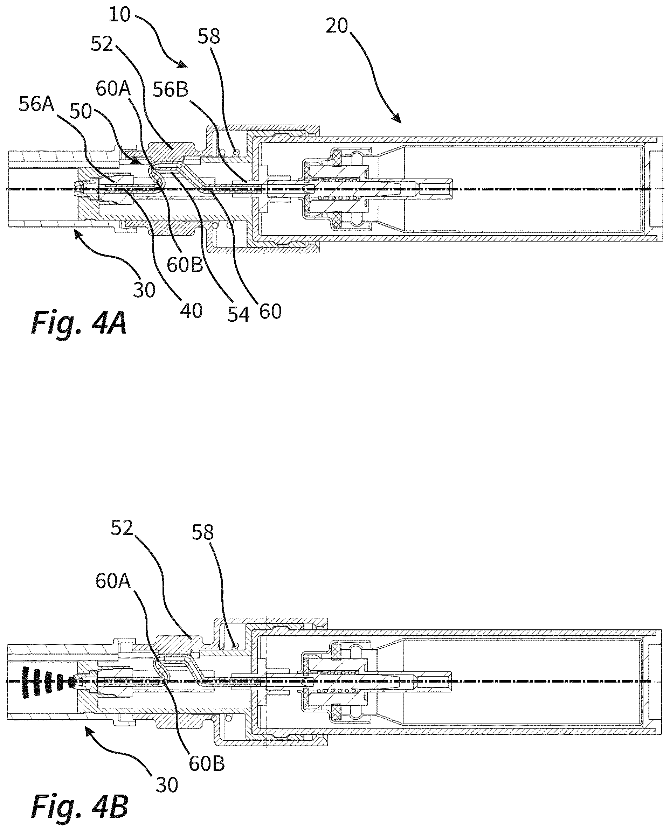

Die Fig. 4A und 4B zeigen eine deutlich abweichende Gestaltung eines Flüssigkeitsspenders 10. Bei dieser Gestaltung ist der Flüssigkeitskanal 40 zwischen dem Flüssigkeitsspeicher 20 und dem Mundstück 30 sowie der dort vorgesehenen Austragdüseneinheit 32 ebenfalls teilweise mit einem Schlauchabschnitt 60 realisiert. Dieser Schlauchabschnitt wird jedoch nicht unmittelbar durch ein Schlauchformungssegment 54 zusammengedrückt. Das Schlauchformungssegment 54, welches hier einen Teilabschnitt des Schlauchabschnitts 60 umgreift, sorgt vielmehr für eine Verlagerung des Schlauchabschnitts 60, durch die zwei Knickstellen 60A, 60B geöffnet und geschlossen werden können. Wird die an einem hülsenförmigen Körper vorgesehene Betätigungsfläche 52 bezogen auf die Fig. 4A und 4B nach rechts verlagert, so verringert sich der Knickradius an den Knickstellen 60A und 60B. Die Folge ist, dass Flüssigkeit durch den Flüssigkeitskanal 40 zur Austragdüseneinheit 32 strömen kann. Wird die Betätigungsfläche 52 losgelassen, so drückt die Rückstellfeder 58 die Betätigungsfläche und auch das Schlauchformungssegment 54 zurück in seine linke Endlage, in der der Knickwinkel und der Krümmungsradius im Bereich der Knickstellen 60A, 60B so verändert ist, dass diese Knickstellen gemeinsam den Durchfluss von Flüssigkeit durch den Flüssigkeitskanal 40 unterbinden.

Die Betätigung muss bei einem Flüssigkeitsspender entsprechend der Fig. 4A und 4B nicht an der Betätigungsfläche 52 erfolgen. Alternativ kann auch vorgesehen sein, dass das Mundstück 30, nicht aber die Austragdüseneinheit 32, in axialer Richtung fest mit dem Bauteil der Betätigungsfläche 52 verbunden ist, so dass ein Drücken des Mundstücks 30 in Richtung des Flüssigkeitsspeichers 20 das Öffnen der Auslassventilanordnung 50 bewirkt.

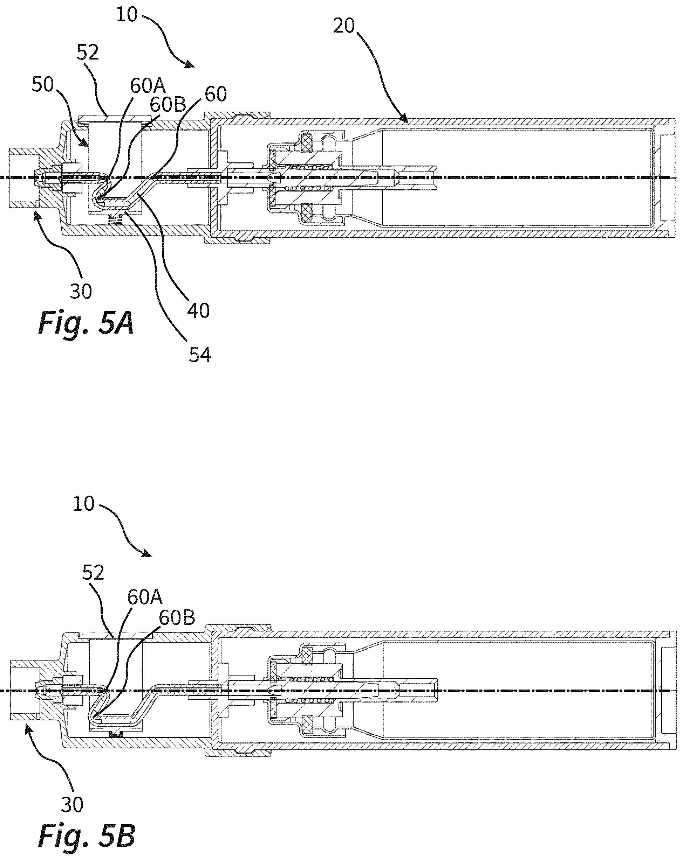

Die Gestaltung der Fig. 5A und 5B ähnelt jener der Fig. 4A und 4B, da auch hier ein Schlauchabschnitt 60 Verwendung findet, der im Bereich zweier Knickstellen 60A, 60B einknicken kann, um hier einen Flüssigkeitsdurchfluss vom Flüssigkeitsspeicher 20 bis zur Austragdüseneinheit 32 zu unterbinden. Anders als bei der Gestaltung der Fig. 4A und 4B wird die entsprechende Verformung des Schlauchabschnitts 60 zum Zwecke des Öffnens und Schließens der durch ihn gebildeten Auslassventilanordnung jedoch nicht durch eine axiale Verlagerung erreicht, sondern durch eine radiale. Die Betätigungsfläche 52 kann hierfür niedergedrückt werden, wodurch sich mittelbar der Krümmungsradius und der Krümmungswinkel im Bereich der Knickstellen 60A, 60B verringern, so dass dann Flüssigkeit durch den Flüssigkeitskanal 40 bis zur Austragdüseneinheit 32 gelangen kann.

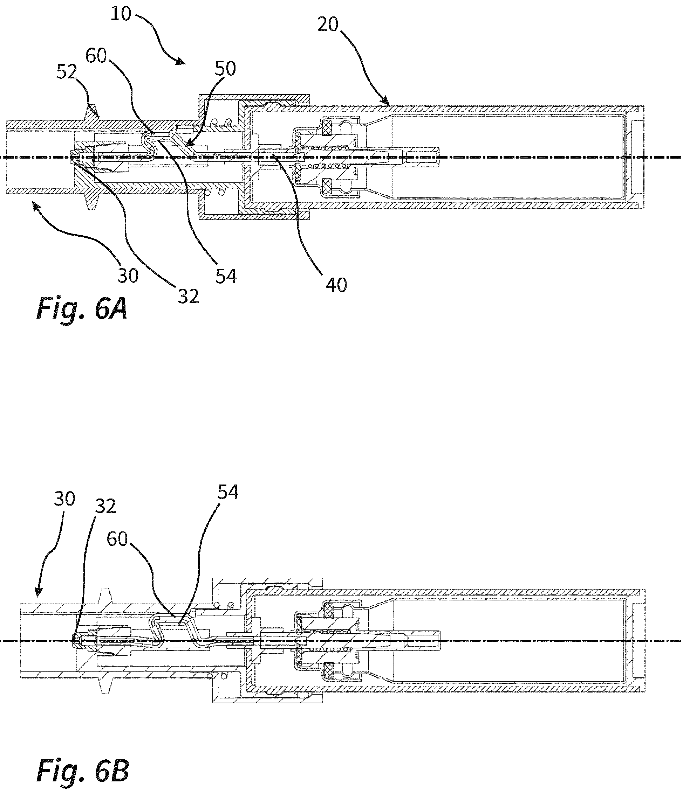

Bei der Gestaltung gemäß Fig. 6A und 6B ist vorgesehen, dass wiederum eine partiell axiale Verschiebung des Schlauchabschnitts 60 erfolgt. Hierfür ist ein einstückiges Bauteil vorgesehen, welches sowohl das Mundstück 30 bildet als auch das Schlauchformungssegment 54. Wenn durch Kraftbeaufschlagung des Flüssigkeitsspeichers 20 gegenüber diesem Bauteil und dem Mundstück 30 der Schlauchabschnitt partiell axial verlagert wird, kommt es an den Knickstellen 60A, 60B zu einer Entspannung, durch die Flüssigkeit bis zur Austragdüseneinheit 32 gelangen kann.

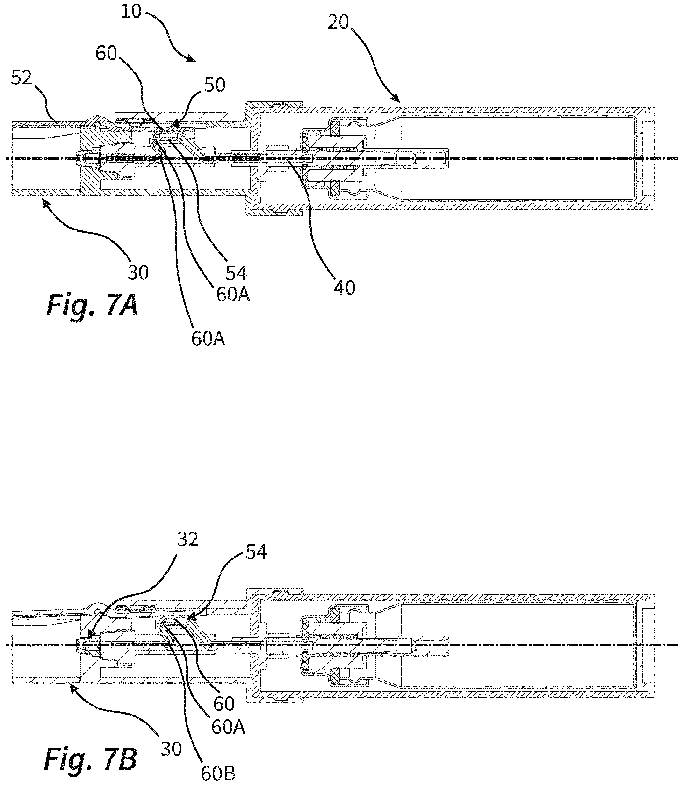

Bei den Fig. 7A und 7B ist ähnlich der Gestaltung der Fig. 3A und 3B ein wippenartiges Element vorgesehen, an dem sowohl die bis zum Mundstück 30 ragende Betätigungsfläche 52 als auch das Schlauchformungssegment 54 vorgesehen sind. Werden die Lippen oder die Zähne durch den Nutzer zusammengedrückt, so verschwenkt dieses Wippenelement gegen den Uhrzeigersinn und gegen die Kraft der Rückstellfeder 58. Hierbei wird das Schlauchformungssegment 54 nach oben verlagert und bewegt somit den Schlauchabschnitt 60 in die Stellung der Fig. 7B, in der Flüssigkeit aus dem Flüssigkeitsspeicher 20 bis zur Austragdüseneinheit 32 gelangen kann.

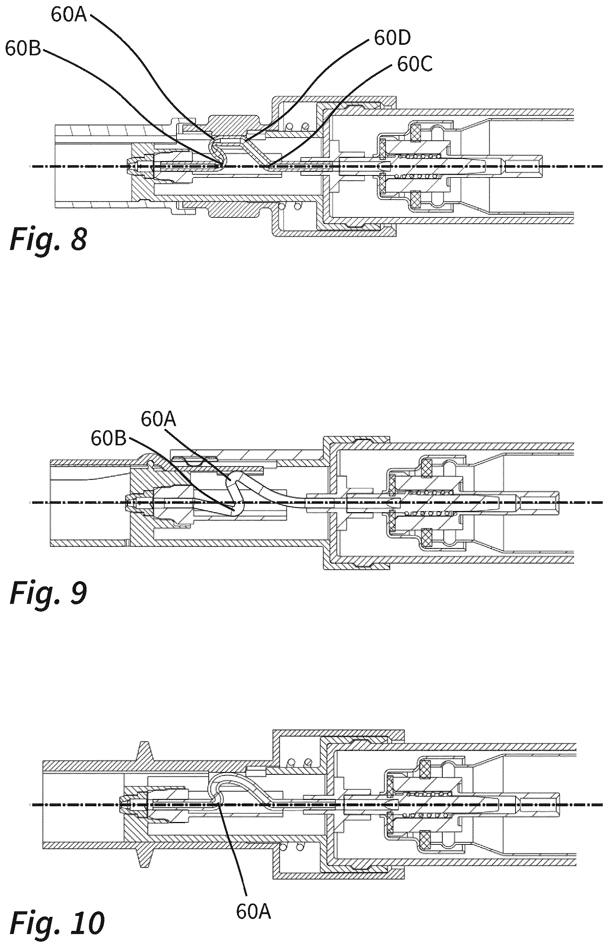

Fig. 8 zeigt nochmals die bei den Gestaltungen der Fig. 4A bis 7B vorgesehene Formgebung des Schlauchabschnitts 60, der hier in etwa eine Parallelogramm-Form beschreibt. Zusätzlich zu den beiden Knickstellen 60A und 60B gibt es dementsprechend noch zwei Krümmungsbereiche 60C und 60D. Wird der Schlauchabschnitt 60 zum Zwecke des Öffnens der Auslassventilanordnung verformt, so wird der Krümmungswinkel an den Knickstellen 60A, 60B verringert, während er in für den Flüssigkeitsfluss unbeachtlicher Weise an den Krümmungsstellen 60C und 60D erhöht wird.

Eine alternative Gestaltung ist der Fig. 9 zu entnehmen. Hier gibt es keine paarweise angeordneten Krümmungsstellen 60C, 60D. Stattdessen ist der Schlauchabschnitt 60 beidseitig der Knickstellen 60A, 60B als Ganzes leicht gekrümmt, um zu erreichen, dass die beiden Enden des Schlauchabschnitts zueinander in fluchtender Ausrichtung sind.

Die Gestaltung der Fig. 10 weist nur eine einzige Knickstelle 60A auf. Im Übrigen ist der Schlauch durch eine keine Knickstelle bildende Krümmung so verlegt, dass er die besagte Knickstelle 60A bildet.

Alle drei Schlauchgestaltungen der Fig. 8 bis 10 könnten bei den Flüssigkeitsspendern der Fig. 4A bis 7B Verwendung finden.

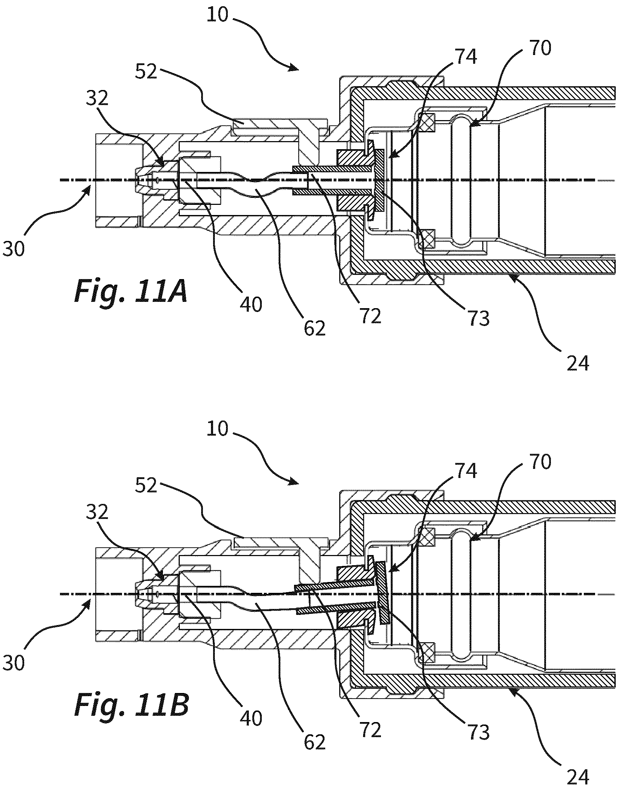

Die Fig. 11A und 11B zeigen eine abweichende Gestaltung einer Auslassventilanordnung. Hier ist vorgesehen, dass die Auslassventilanordnung 70, die es gestattet, Flüssigkeit zur Austragdüseneinheit 32 strömen zu lassen, in Art eines Kippventils 74 gestaltet ist. Dieses Kippventil 74 ist bei den hier vorgestellten Ausführungsbeispielen fest mit einer Flüssigkeitskartusche 24 verbunden, könnte jedoch auch getrennt von der Flüssigkeitskartusche 24 ausgebildet sein. Das Kippventil 74 weist einen Auslassstutzen 72 auf, der an seinem in den Figuren rechten Ende in einen Ventilteller 73 übergeht. Wird der Auslassstutzen 72 verkippt, so verkippt hiermit auch die Ventilfläche 73 und gestattet den Austritt von Flüssigkeit in den Auslassstutzen 72 und von hier durch einen Schlauchabschnitt 62 bis zur Austragdüseneinheit 32.

Bei der Gestaltung der Fig. 11A und 11B ist vorgesehen, dass über eine Betätigungsfläche 52, die zur manuellen Betätigung vorgesehen ist, eine Auslenkung des Auslassstutzens 72 stattfindet, wie es in Fig. 11B verdeutlicht ist. Der Schlauchabschnitt 62 gewährleistet dabei, dass die Flüssigkeit, die durch den Auslassstutzen 72 ausströmt, zur Austragdüseneinheit 32 geleitet wird.

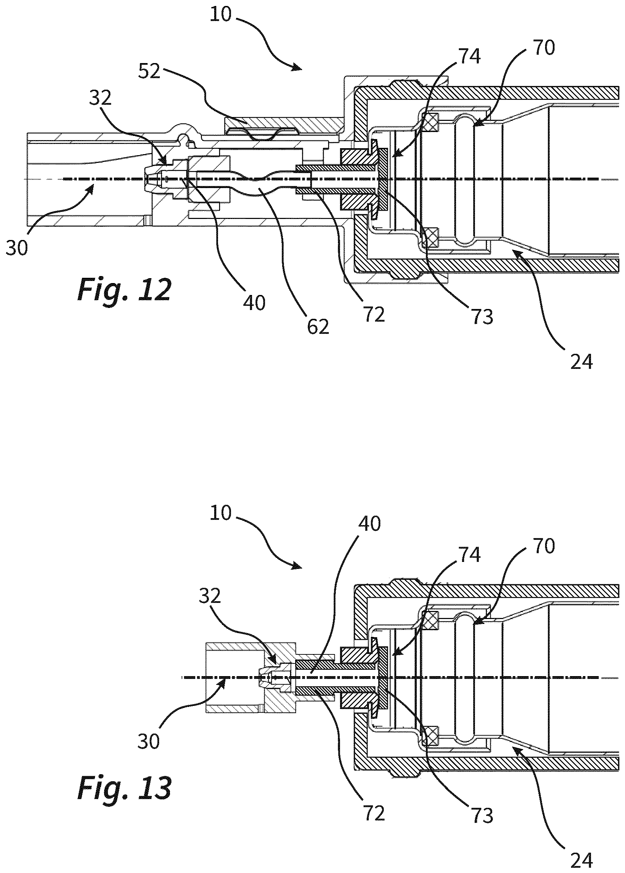

Bei der Variante der Fig. 12 ist ebenfalls ein Kippventil 74 gleicher Art vorgesehen. Dieses wird jedoch in diesem Fall durch eine Betätigung geöffnet, die mittels des Munds, also mittels Zusammendrücken der Zähne oder der Lippen, verlagert wird. Die Betätigungsfläche 52 ist Teil eines Wippenelements, dessen der Betätigungsfläche 52 gegenüberliegendes Ende beim Niederdrücken der Betätigungsfläche 52 das Verkippen des Auslassstutzens 72 bewirkt.

Eine besonders einfache Gestaltung zeigt Fig. 13. Auch hier ist ein Kippventil 74 vorgesehen. Dieses ist in diesem Falle jedoch fest mit dem Mundstück 30 verbunden. Ein Öffnen des Kippventils erfolgt hier dadurch, dass der Nutzer den Flüssigkeitsspeicher 20 leicht gegenüber dem Mundstück 30 verkippt. Die ausströmende Flüssigkeit gelangt durch den Auslassstutzen 72 hindurch bis zum Mundstück 30 und der dort vorgesehenen Austragdüseneinheit.

## Patentansprüche

1. Spender (10) zur Abgabe einer nikotin- und/oder cannabishaltigen Flüssigkeit mit den folgenden Merkmalen:
a. der Spender (10) weist einen Flüssigkeitsspeicher (20) zur Lagerung der Flüssigkeit vor dem Austrag auf, und
b. der Spender weist ein Mundstück (30) auf, durch welches die Flüssigkeit abgegeben werden kann, und
c. der Flüssigkeitsspeicher (20) ist durch einen Flüssigkeitskanal (40) mit dem Mundstück (30) verbunden, und
d. der Spender (10) weist eine Auslassventilanordnung (50) auf, mittels derer der Flüssigkeitskanal (40) geschlossen und geöffnet werden kann,
**gekennzeichnet durch** das folgende Merkmal:
e. die Auslassventilanordnung (70) weist ein Kippventil (74) auf, welches über einen kippbeweglichen Auslassstutzen (72) verfügt, der von dem Flüssigkeitskanal (40) durchdrungen ist, wobei in Abhängigkeit der Schwenkstellung des Auslassstutzens (72) das Kippventil (74) geöffnet bzw. geschlossen ist.

2. Spender (10) nach Anspruch 1 mit dem folgenden zusätzlichen Merkmal:
a. das Mundstück (30) ist gegenüber dem Flüssigkeitsspeicher (20) beweglich ausgebildet und mit dem Auslassstutzen (72) der Auslassventilanordnung (70) derart gekoppelt, dass eine Bewegung des Mundstücks (30) gegenüber dem Flüssigkeitsspeicher (20) den Auslassstutzen (72) verschwenkt.

3. Spender (10) nach Anspruch 2 mit dem folgenden zusätzlichen Merkmal:
a. das Mundstück (30) ist fest mit dem Auslassstutzen (72) verbunden.

4. Spender (10) nach Anspruch 1, 2 oder 3 mit den folgenden zusätzlichen Merkmalen:
a. die Auslassventilanordnung (70) weist eine Betätigungsfläche (52) an einer Außenseite des Spenders auf, die gegenüber dem Flüssigkeitsspeicher (20) beweglich ist, und
b. die Betätigungsfläche (52) ist mit dem Auslassstutzen (72) wirkgekoppelt, so dass durch Kraftbeaufschlagung der Betätigungsfläche (52) der Auslassstutzen (72) verschwenkt werden kann.

5. Spender (10) nach einem der vorstehenden Ansprüche mit dem folgenden zusätzlichen Merkmal:
a. zwischen dem Auslassstutzen (72) und dem Mundstück (30) ist ein Schlauchabschnitt (62) vorgesehen.

6. Spender (10) nach einem der vorstehenden Ansprüche mit dem folgenden zusätzlichen Merkmal:
a. der Flüssigkeitsspeicher (20) verfügt über eine Flüssigkeitskartusche (24), wobei das Kippventil (74) an einem Auslass dieser Flüssigkeitskartusche (24) vorgesehen ist.

7. Spender (10) nach Anspruch 6 mit dem folgenden zusätzlichen Merkmal:
a. der Flüssigkeitsspeicher (20) weist eine Aufnahme (22) zur vorzugsweise wechselbaren Aufnahme der Flüssigkeitskartusche (24) auf.

8. Spender (10) nach einem der vorstehenden Ansprüche mit dem folgenden zusätzlichen Merkmal:
a. der Spender (10) weist eine Austragdüseneinheit (32) auf, durch die Flüssigkeit in das Mundstück (30) abgegeben werden kann,
insbesondere mit mindestens einem der folgenden zusätzlichen Merkmale:
b. die Austragdüseneinheit (32) weist eine Düsenplatte (34) auf, in der eine Mehrzahl von Düsenöffnungen vorgesehen sind, und/oder
c. es sind mindestens 10 Düsenöffnungen vorgesehen, und/oder
d. die Düsenöffnungen weisen an ihrer engsten Stelle einen lichten Querschnitt von nicht mehr als 250 µm² auf.

9. Spender (10) nach Anspruch 8 mit dem folgenden zusätzlichen Merkmal:
a. die Austragdüseneinheit (32) verfügt über eine Mehrzahl von Düsenöffnungen.

10. Spender (10) nach einem der vorstehenden Ansprüche mit mindestens dem folgenden zusätzlichen Merkmal:
a. der Spender (10) weist eine Rückstellfeder (58) auf, mittels derer die Betätigungsfläche (52) in Richtung einer unbetätigten Endlage kraftbeaufschlagt ist.

11. Spender (10) nach einem der vorstehenden Ansprüche mit den folgenden zusätzlichen Merkmalen:
a. der Spender (10) weist eine in Richtung der Haupterstreckungsrichtung (2) erstreckte längliche Formgebung auf, und/oder
b. der Flüssigkeitsspeicher (20) des Spenders (10) erstreckt sich in Richtung der Haupterstreckungsrichtung (2), und/oder
c. das Mundstück (30) des Spenders (10) erstreckt sich in Richtung der Haupterstreckungsrichtung (2).

12. Spender (10) nach einem der vorstehenden Ansprüche mit mindestens einem der folgenden zusätzlichen Merkmale:
a. das Mundstück (30) weist eine röhrenförmige Gestalt mit einer Auslassöffnung (36) auf, wobei die Auslassöffnung (36) vorzugsweise einen lichten Querschnitt von mindestens 20 mm² aufweist, und/oder
b. das Mundstück (30) weist einen Lufteinlasskanal (38) auf, der vorzugsweise als radial ausgerichtete Öffnung (38) in einer Wandung des Mundstücks (30) vorgesehen ist, und/oder
c. der Schlauchabschnitt (60) ist aus einem elastischen Material gefertigt, insbesondere aus Polyethylen, Polypropylen oder einem Polyethylen und/oder Polypropylen enthaltenden Material, und/oder
d. der Schlauchabschnitt (60) weist zumindest abschnittsweise und vorzugsweise in einem Knickbereich einen Innendurchmesser von 2,0 mm oder weniger, vorzugsweise von 1,0 mm oder weniger auf, und/oder
e. der Schlauchabschnitt (60) weist zumindest abschnittsweise und vorzugsweise in einem Knickbereich eine Wandungsstärke von weniger als 1,0 mm beträgt, insbesondere von 0,5 mm oder weniger auf, und/oder
f. der Schlauchabschnitt (60) weist zumindest abschnittsweise und vorzugsweise in einem Knickbereich eine Wandungsstärke von mindestens als 0,1 mm oder mehr, insbesondere von 0,3 mm oder mehr auf.

13. Spender (10) nach einem der vorstehenden Ansprüche mit dem folgenden zusätzlichen Merkmal:
a. der Flüssigkeitsspeicher (20) ist mit einer nikotin- und/oder cannabishaltigen Flüssigkeit befüllt.

14. Spender (10) nach einem der vorstehenden Ansprüche mit dem folgenden zusätzlichen Merkmal:
a. der Flüssigkeitsspeicher (20) ist als Druckspeicher ausgebildet, in dem die Flüssigkeit unter Druck gelagert werden kann.

## Claims

1. Dispenser (10) for discharging a nicotine-containing and/or cannabis-containing liquid, having the following features:
a. the dispenser (10) has a liquid store (20) for storing the liquid before the discharge, and
b. the dispenser has a mouthpiece (30), through which the liquid can be discharged, and
c. the liquid store (20) is connected to the mouthpiece (30) by way of a liquid channel (40), and
d. the dispenser (10) has an outlet valve arrangement (50), by means of which the liquid channel (40) can be closed and opened,
**characterized by** the following feature:
e. the outlet valve arrangement (70) has a tilt valve (74) which has a tiltably movable outlet nozzle (72) which is penetrated by the liquid channel (40), the tilt valve (74) being opened and closed in a manner which is dependent on the pivoting position of the outlet nozzle (72).

2. Dispenser (10) according to Claim 1, having the following additional feature:
a. the mouthpiece (30) is of movable configuration with respect to the liquid store (20), and is coupled to the outlet nozzle (72) of the outlet valve arrangement (70) in such a way that a movement of the mouthpiece (30) with respect to the liquid store (20) pivots the outlet nozzle (72).

3. Dispenser (10) according to Claim 2, having the following additional feature:
a. the mouthpiece (30) is connected fixedly to the outlet nozzle (72).

4. Dispenser (10) according to Claim 1, 2 or 3, having the following additional features:
a. the outlet valve arrangement (70) has an actuating surface (52) on an outer side of the dispenser, which actuating surface can be moved with respect to the liquid store (20), and
b. the actuating surface (52) is operatively coupled to the outlet nozzle (72), with the result that the outlet nozzle (72) can be pivoted by way of loading the actuating surface (52) with force.

5. Dispenser (10) according to one of the preceding claims, having the following additional feature:
a. a hose portion (62) is provided between the outlet nozzle (72) and the mouthpiece (30).

6. Dispenser (10) according to one of the preceding claims, having the following additional feature:
a. the liquid store (20) has a liquid cartridge (24), the tilt valve (74) being provided at an outlet of this liquid cartridge (24).

7. Dispenser (10) according to Claim 6, having the following additional feature:
a. the liquid store (20) has a receptacle (22) for receiving the liquid cartridge (24) in a preferably replaceable manner.

8. Dispenser (10) according to one of the preceding claims, having the following additional feature:
a. the dispenser (10) has a discharge nozzle unit (32), through which liquid can be discharged into the mouthpiece (30),
in particular having at least one of the following additional features:
b. the discharge nozzle unit (32) has a nozzle plate (34), in which a plurality of nozzle openings are provided, and/or
c. at least 10 nozzle openings are provided, and/or
d. the nozzle openings have an inside cross section of no more than 250 µm² at their narrowest point.

9. Dispenser (10) according to Claim 8, having the following additional feature:
a. the discharge nozzle unit (32) has a plurality of nozzle openings.

10. Dispenser (10) according to one of the preceding claims, having at least the following additional feature:
a. the dispenser (10) has a restoring spring (58), by means of which the actuating surface (52) is loaded with force in the direction of a non-actuated end position.

11. Dispenser (10) according to one of the preceding claims, having the following additional features:
a. the dispenser (10) has an elongate shape which extends in the direction of the main direction of extent (2), and/or
b. the liquid store (20) of the dispenser (10) extends in the direction of the main direction of extent (2), and/or
c. the mouthpiece (30) of the dispenser (10) extends in the direction of the main direction of extent (2).

12. Dispenser (10) according to one of the preceding claims, having at least one of the following additional features:
a. the mouthpiece (30) has a tubular design with an outlet opening (36), the outlet opening (36) preferably having an inside cross section of at least 20 mm², and/or
b. the mouthpiece (30) has an air inlet channel (38) which is preferably provided as a radially oriented opening (38) in a wall of the mouthpiece (30), and/or
c. the hose portion (60) is manufactured from an elastic material, in particular from polyethylene, polypropylene or a material which contains polyethylene and/or polypropylene, and/or
d. the hose portion (60) has an internal diameter of 2.0 mm or less, preferably of 1.0 mm or less, at least in sections and preferably in a bending region, and/or
e. the hose portion (60) has a wall thickness of less than 1.0 mm, in particular of 0.5 mm or less, at least in sections and preferably in a bending region, and/or
f. the hose portion (60) has a wall thickness of at least 0.1 mm or more, in particular of 0.3 mm or more, at least in sections and preferably in a bending region.

13. Dispenser (10) according to one of the preceding claims, having the following additional feature:
a. the liquid store (20) is filled with a nicotine-containing and/or cannabis-containing liquid.

14. Dispenser (10) according to one of the preceding claims, having the following additional feature:
a. the liquid store (20) is configured as a pressurized store, in which the liquid can be stored under pressure.

## Revendications

1. Distributeur (10) destiné à distribuer un liquide contenant de la nicotine et/ou du cannabis avec les caractéristiques suivantes :
a. le distributeur (10) comporte un réservoir de liquide (20) pour stocker le liquide avant de le décharger, et
b. le distributeur comporte un embout (30), par lequel le liquide peut être distribué, et
c. le réservoir de liquide (20) est relié à l'embout (30) par un canal de liquide (40), et
d. le distributeur (10) comporte un ensemble de soupape de sortie (50), au moyen duquel le canal de liquide (40) peut être fermé et ouvert,
**caractérisé par** la caractéristique suivante :
e. l'ensemble de soupape de sortie (70) comporte une soupape à basculement (74), laquelle dispose d'une tubulure de sortie (72) mobile par basculement, qui est traversée par le canal de liquide (40), la soupape à basculement (74) étant ouverte ou fermée en fonction de la position de pivotement de la tubulure de sortie (72).

2. Distributeur (10) selon la revendication 1 avec la caractéristique supplémentaire suivante :
a. l'embout (30) est réalisé de manière mobile par rapport au réservoir de liquide (20) et est couplé à la tubulure de sortie (72) de l'ensemble de soupape de sortie (70) de telle manière qu'un déplacement de l'embout (30) fait pivoter la tubulure de sortie (72) par rapport au réservoir de liquide (20).

3. Distributeur (10) selon la revendication 2 avec la caractéristique supplémentaire suivante :
a. l'embout (30) est relié de manière solidaire à la tubulure de sortie (72).

4. Distributeur (10) selon la revendication 1, 2 ou 3, avec les caractéristiques supplémentaires suivantes :
a. l'ensemble de soupape de sortie (70) comporte une surface d'actionnement (52) sur un côté extérieur du distributeur, qui est mobile par rapport au réservoir de liquide (20), et
b. la surface d'actionnement (52) est couplée de manière active à la tubulure de sortie (72) de telle sorte que la tubulure de sortie (72) peut être pivotée par l'action d'une force appliquée sur la surface d'actionnement (52).

5. Distributeur (10) selon l'une des revendications précédentes, avec la caractéristique supplémentaire suivante :
a. un tronçon de tuyau flexible (62) est prévu entre la tubulure de sortie (72) et l'embout (30).

6. Distributeur (10) selon l'une des revendications précédentes, avec la caractéristique supplémentaire suivante :
a. le réservoir de liquide (20) dispose d'une cartouche de liquide (24), la soupape à basculement (74) étant prévue sur une sortie de la cartouche de liquide (24).

7. Distributeur (10) selon la revendication 6 avec la caractéristique supplémentaire suivante :
a. le réservoir de liquide (20) comporte un logement (22) destiné à recevoir de préférence de manière interchangeable la cartouche de liquide (24).

8. Distributeur (10) selon l'une des revendications précédentes, avec la caractéristique supplémentaire suivante :
a. le distributeur (10) comporte une unité de buse de décharge (32), par laquelle le liquide peut être distribué dans l'embout (30),
en particulier avec au moins une des caractéristiques supplémentaires suivantes :
b. l'unité de buse de décharge (32) comporte une plaque à buses (34), dans laquelle sont prévues une multitude d'ouvertures de buse, et/ou
c. au moins 10 ouvertures de buse sont prévues, et/ou
d. les ouvertures de buse comportent, sur leur emplacement le plus étroit, une section transversale intérieure ne dépassant pas 250 µm².

9. Distributeur (10) selon la revendication 8 avec la caractéristique supplémentaire suivante :
a. l'unité de buse de décharge (32) dispose d'une multitude d'ouvertures de buse.

10. Distributeur (10) selon l'une des revendications précédentes, avec la caractéristique supplémentaire suivante :
a. le distributeur (10) comporte un ressort de rappel (58), au moyen duquel la surface d'actionnement (52) est soumise à l'action d'une force en direction d'une position terminale non actionnée.

11. Distributeur (10) selon l'une des revendications précédentes, avec les caractéristiques supplémentaires suivantes :
a. le distributeur (10) présente un façonnage allongé s'étendant en direction de la direction d'extension principale (2), et/ou
b. le réservoir de liquide (20) du distributeur (10) s'étend en direction de la direction d'extension principale (2), et/ou
c. l'embout (30) du distributeur (10) s'étend en direction de la direction d'extension principale (2).

12. Distributeur (10) selon l'une des revendications précédentes, avec au moins une des caractéristiques supplémentaires suivantes :
a. l'embout (30) présente une forme tubulaire avec une ouverture de sortie (36), l'ouverture de sortie (36) présentant de préférence une section transversale intérieure d'au moins 20 mm², et/ou
b. l'embout (30) comporte un canal d'entrée d'air (38), qui est prévu de préférence en tant qu'une ouverture (38) orientée radialement dans une paroi de l'embout (30), et/ou
c. le tronçon de tuyau flexible (60) est produit à partir d'un matériau élastique, en particulier à partir de polyéthylène, de polypropylène ou d'un matériau contenant du polyéthylène et/ou du polypropylène, et/ou
d. le tronçon de tuyau flexible (60) présente au moins par endroits et de préférence dans une zone de pliage un diamètre intérieur inférieur ou égal à 2,0 mm, de préférence inférieur ou égal à 1,0 mm, et/ou
d. le tronçon de tuyau flexible (60) présente au moins par endroits et de préférence dans une zone de pliage une épaisseur de paroi inférieure à 1,0 mm, en particulier inférieure ou égale à 0,5 mm, et/ou
f. le tronçon de tuyau flexible (60) présente au moins par endroits et de préférence dans une zone de pliage une épaisseur de paroi supérieure ou égale à 0,1 mm, en particulier supérieure ou égale à 0,3 mm.

13. Distributeur (10) selon l'une des revendications précédentes, avec la caractéristique supplémentaire suivante :
a. le réservoir de liquide (20) est rempli d'un liquide contenant de la nicotine et/ou du cannabis.

14. Distributeur (10) selon l'une des revendications précédentes, avec la caractéristique supplémentaire suivante :
a. le réservoir de liquide (20) est réalisé comme un réservoir sous pression, dans lequel le liquide peut être stocké sous pression.
